# EUROPÄISCHE PATENTSCHRIFT

(11) **EP 1 175 918 B1**
(45) Veröffentlichungstag und Bekanntmachung des Hinweises auf die Patenterteilung: **01.09.2004**
(21) Anmeldenummer: 01113862.5
(22) Anmeldetag: 07.06.2001
(51) Int. Cl.: A61M 5/31, B05D 3/02, B05D 7/22

(54) **Verfahren und Vorrichtung zum Auftragen einer hitzefixierten Gleitmittelschicht**
Process and device for application of a heat secured lubricant layer
Procédé et dispositif d'application à chaud d'une couche de lubrifiant

(30) Priorität: 28.07.2000 DE 10036832
(43) Veröffentlichungstag der Anmeldung: 30.01.2002
(73) Patentinhaber: Schott Glas, 55122 Mainz (DE); CARL-ZEISS-STIFTUNG trading as SCHOTT GLAS, 55122 Mainz (DE)
(72) Erfinder: Heinz, Jochen, Dr., 24149 Kiel (DE); Spallek, Michael, Dr., 55218 Ingelheim (DE)
(74) Vertreter: Fuchs Mehler Weiss & Fritzsche

(56) Entgegenhaltungen:
- EP-A- 0 675 315
- DE-A- 19 753 766
- US-A- 3 804 663

## Beschreibung

Die Erfindung bezieht sich auf ein Verfahren zum Aufbringen einer hitzefixierten Gleitmittelschicht auf die Innenwandung von zylindrischen medizinischen Behältern, die an einem Ende eine sich verjüngende Austrittsöffnung aufweisen und die am anderen Ende mit einem im Behälter als Kolben verschiebbaren Elastomerstopfen verschließbar sind.

Die Erfindung bezieht sich ferner auf eine Vorrichtung zur Durchführung dieses Verfahrens.

Bei Behältern für medizinische Zwecke, insbesondere Pharmapackmitteln oder Behälter für Diagnostika, bestehend aus länglichen Hohlkörpern, ist es notwendig, daß ein in den Hohlraum eingebrachter elastomerer Verschluß, z.B. ein Kolben bzw. ein Kolbenstopfen, geschmeidig an der Innenwandung des Hohlkörpers gleitet. Typische Beispiele sind Spritzenzylinder für vorfüllbare Spritzen, Spritzenkörper von Zylinderampullen und Karpulen, aber auch Kolbenbüretten in der chemischen Analytik. Um diesem Problem gerecht zu werden, wird typischerweise auf die Innenwandung des Hohlkörpers eine Silikon-Gleitschicht aufgebracht.

Von besonderer Bedeutung sind dabei die vorfüllbaren Spritzen. Vorfüllbare Spritzen aus Glas sind hinlänglich aus zahlreichen Schriften bekannt. Dabei handelt es sich um Spritzenkörper aus Glas, die keine integrierte Nadel aufweisen. Glasspritzen mit eingeklebter Nadel sind ebenfalls notorisch bekannt.

Beide Arten von vorfüllbaren Spritzen aus Glas benötigen zwischen dem beweglichen Elastomerkolben und dem Glas-Spritzenzylinder ein Gleitmittel. Hierzu wird bevorzugt Silikonöl eingesetzt. Dieses Silikonöl wird üblicherweise beim Waschen auf das Innere der Spritzenzylinder aufgetragen, wobei gravierende Unterschiede bei der Verarbeitung von Spritzenkörpern mit und ohne integrierte Nadel vorhanden sind. Da die Nadel typischerweise mit nicht ausreichend wärmebeständigen Klebern, wie z.B. Epoxydharz oder anderen nur begrenzt hitzebeständigen Klebern fixiert wird, ergeben sich insoweit unterschiedliche Verarbeitungsbedingungen.

Bei Fertigspritzen mit eingeklebter Kanüle ist nur eine maximale Verarbeitungstemperatur von 130°C an der Klebestelle möglich. Eine dauerhafte Gleitmittelbeschichtung ist jedoch nur dann gewährleistet, wenn das Gleitmittel, vorteilhafterweise Silikon, bei Temperaturen um 300°C aufgebracht wird.

Daher ist es bekannt, daß zunächst der Glas-Spritzenzylinder mit einer bei ca. 300°C behandelten Silikonbeschichtung versehen wird und anschließend ein nadeltragendes Ansatzstück mit dem Spritzenkörper aus Glas verbunden wird. Diese Maßnahme ist jedoch mit zusätzlichem Montageaufwand und damit Kosten verbunden, was sich insbesondere dadurch nachteilig bemerkbar macht, weil es sich hierbei um ein Massenprodukt handelt.

Bei Spritzen mit eingeklebter Nadel besteht zudem beim Aufbringen der Silikon-Gleitschicht das Risiko, daß der oftmals sehr enge Nadelkanal ebenfalls mit Silikon-Gleitmittel gefüllt wird, was dann im Gebrauchsfall leicht zur unzulässigen Injektion von Silikonöl führen kann.

Ähnlich liegt die Problematik bei vorfüllbaren Kunststoffspritzen. Die DE 197 53 766 A1 beschreibt dabei ein Verfahren zur Innensilikonisierung von Behältern für medizinische Zwecke, bestehend aus einem länglichen Kunststoff-Hohlkörper, insbesondere Spritzenzylinder, unter Verwendung spezieller reaktiver Silikonöle, die mit Licht ausgehärtet werden. Dieses Verfahren vermeidet die sonst üblichen Vorkonditionierungsschritte der Innenoberfläche, z.B. durch eine Plasmabehandlung.

Der Erfindung liegt die Aufgabe zugrunde, das eingangs bezeichnete Verfahren zum Aufbringen einer hitzefixierten Gleitmittelschicht auf die Innenwandung von zylindrischen medizinischen Behältern, die an einem Ende eine sich verjüngende Austrittsöffnung aufweisen, und die am anderen Ende mit einem im Behälter als Kolben verschiebbaren Elastomerstopfen verschließbar sind, so zu führen, bzw. die zugehörige Vorrichtung so auszubilden, daß eine thermische Fixierung des Gleitmittels bei sehr hohen Temperaturen möglich ist, ohne daß temperaturempfindliche Teile am austrittsseitigen Ende des Behälters dabei geschädigt werden.

Hinsichtlich des Verfahrens gelingt die Lösung gemäß der Erfindung mit den Schritten:
- Auftragen eines hitzefixierbaren Gleitmittels auf die Innenwandung des Behälters mit bekannter Technologie,
- Vergleichmäßigen des aufgetragenen Gleitmittels zu einer Schicht, und
- Hitzefixieren der Gleitmittelschicht durch eine Infrarotstrahlung selektiv im zylindrischen Mantelbereich des Behälters mit hohen Temperaturen oberhalb der maximalen betrieblichen Einsatztemperatur des Behälters.

Hinsichtlich der Vorrichtung zur Durchführung des Verfahrens gelingt die Lösung der Aufgabe erfindungsgemäß mit
- einer Einrichtung zum Auftragen des hitzefixierbaren Gleitmittels aus einem Vorratsbehälter auf die Innenwandung des Behälters,
- einer Einrichtung zum Vergleichmäßigen der aufgetragenen Gleitmittelschicht, und
- einer in das Innere des Behälters einbringbaren stabförmigen Infrarot-Strahlungsquelle, die kopfseitig eine Strahlungsabschirmung zum Fernhalten der Strahlung von der Austrittsöffnung des Behälters besitzt.

Durch die selektive Erwärmung der Behälterwandung mittels der Infrarotstrahlung kann die dort aufgetragene Gleitmittelschicht auf einfache Weise und mit hohen Temperaturen sicher fixiert werden, wogegen die Austrittsöffnung des Behälters gleitmittelfrei bleibt und auch die Verklebung einer Injektionsnadel unbeeinträchtigt bleibt. Gleichzeitig findet durch die hohen Temperaturen eine Reduzierung der Keimzahl und von Endotoxinen statt. Ferner sind bei dem erfindungsgemäßen Verfahren mit Vorteil keine Vorkonditonierungsschritte der Oberfläche, z.B. durch Plasmabehandlung, notwendig.

Das erfindungsgemäße Verfahren und die erfindungsgemäße Vorrichtung eignen sich daher mit Vorteil zur Aufbringung von hitzefixierten Gleitmittelschichten, insbesondere silikonartigen Schichten, sowohl auf Spritzenkörpern aus Glas oder Kunststoff ohne integrierte Nadel als auch auf Glas- oder Kunststoffspritzenkörpern mit integrierter Nadel.

Vorzugsweise wird bei dem erfindungsgemäßen Verfahren als Gleitmittel Silikonöl verwendet, sei es als reaktives oder nicht reaktives Silikonöl oder sei es eine Mischung aus beiden Silikonöl-Typen. Zur Anwendung können auch andere hitzefixierbare Gleitmittel.

Gemäß einer Ausgestaltung der Erfindung erfolgt das Auftragen des Gleitmittels vorzugsweise durch Aufsprühen, was auf sehr einfache Weise zu einer relativ gleichförmig benetzenden Gleitmittelschicht führt. Aber auch andere bekannte Methoden zum Aufbringen von Gleitmitteln, insbesondere Silikonölen, sind denkbar.

Der Temperaturbereich der Hitzefixierung ist u.a. abhängig davon, ob ein Glas- oder Kunststoffbehälter zu beschichten ist. Bei einem Glasbehälter liegt der Temperaturbereich vorzugsweise bei ca. 300°C, wogegen im Fall eines Kunststoffbehälters die Hitzefixierung in einem Temperaturbereich erfolgt, der um ca. 50°C oberhalb der maximalen betrieblichen Einsatztemperatur des Kunststoffbehälters liegt.

Zum selektiven Bestrahlen der Innenwand des Behälters besitzt die stabförmige Infrarotstrahlungsquelle als Strahlungsabschirmung vorzugsweise eine kopfseitige Abdeckung in Form einer zylindrischen Kappe. Alternativ kann die Abschirmung auch die Form eines pilzförmigen Körpers haben, der mit seinem Stiel in der Kopf-Stirnseite befestigt ist und der mittels seines Schirmes die Infrarotstrahlung von der Austrittsöffnung des Behälters fernhält. Andere Formen der Abschirmung sind denkbar.

Anhand eines in den Zeichnungen dargestellten Ausführungsbeispieles wird die Erfindung näher erläutert.

Es zeigen:
- Figur 1: in einer schematischen Längsschnitt-Darstellung das Auftragen einer Gleitmittelschicht in einem Spritzenkörper durch Aufsprühen eines hitzefixierbaren Gleitmittels auf die Innenwandung des Spritzenkörpers,
- Figur 2: in einer prinzipiellen Längsschnitt-Darstellung das Hitzefixieren der gemäß Figur 1 aufgetragenen und danach vergleichmäßigten Gleitmittelschicht mittels eines stabförmigen Infrarotstahles, der in das Innere des Spritzenkörpers eingebracht ist, und
- Figur 3: in einer Ausschnitt-Darstellung eine vergrößerte Ansicht des Kopfes des stabförmigen Infrarotstrahlers nach Figur 2 mit einer Abschirmung der kopfseitig austretenden Infrarotstrahlen, und zwar im Figurenteil A mit einer auf das Ende aufgebrachten zylindrischen Abdeckkappe und im Figurenteil B in Form eines pilzförmigen Körpers, der an der Stirnseite des Kopfes mit seinem Stiel angebracht ist.

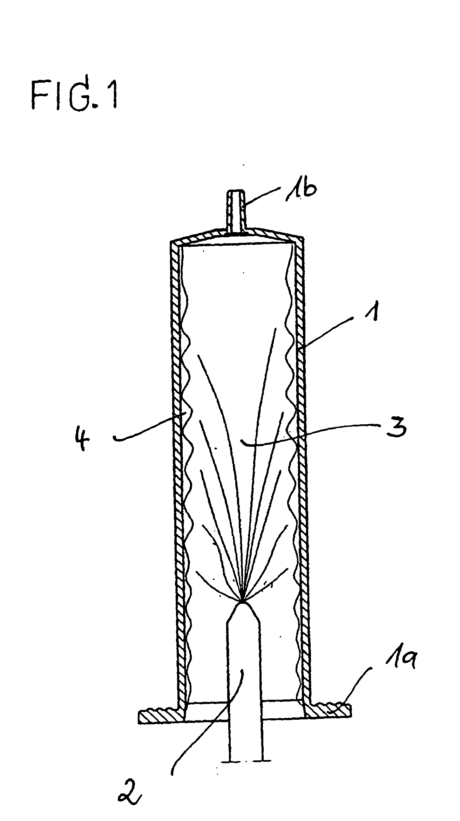

Die Figur 1 zeigt einen Spritzenkörper 1 aus Glas oder Kunststoff mit angeformter Griffleiste 1a und einem Luer-Konus 1b am Austrittsende. In dem Spritzenkörper ist eine Sprühdüse 2 zum Aufsprühen von üblichen Silikonölemulsionen oder reaktiven thermisch fixierbaren Silikonen 3 auf die zu beschichtende Innenoberfläche des Spritzenzylinders 1 eingeführt. Auch Mischungen dieser Silikonöltypen können aufgesprüht werden.

Das Aufsprühen nach Figur 1 ist nur eine der zur Verfügung stehenden Möglichkeiten. Die Silikonölemulsionen bzw. reaktiven Silikone können auch durch ein Schwämmchen oder mittels eines bewegten Kolbens, der entsprechende Austrittsöffnungen hat, oder durch andere bekannte Methoden, wie Aufbürsten, etc. aufgetragen werden.

Im nächsten, nicht dargestellten Arbeitsschritt, wird der noch unfixierte Gleitmittelfilm 4, z.B. durch Abstreifen mittels eines entsprechenden Werkzeuges, möglichst uniform auf der Innenoberfläche des Spritzenkörpers 1 verteilt.

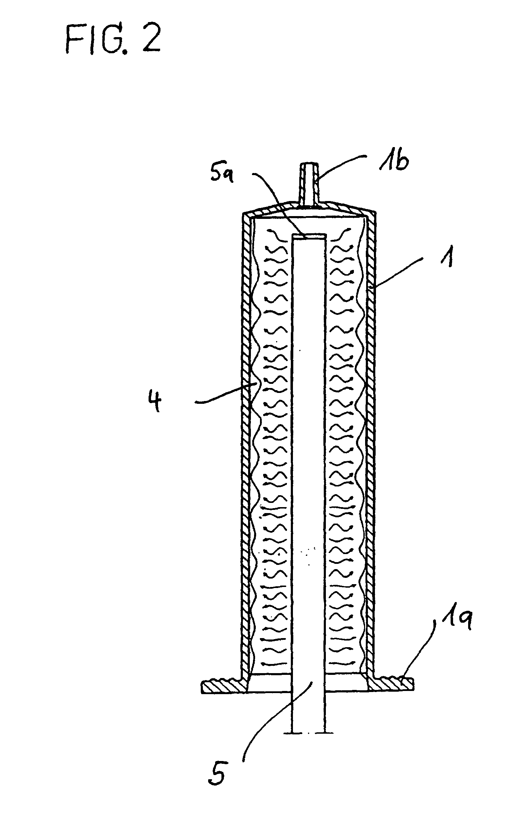

Danach findet die Fixierung des noch mobilen Gleitmittelfilms 4 auf der Oberfläche durch eine selektiv durchgeführte Hitzebehandlung statt. Diese Hitzebehandlung erfolgt gemäß Figur 2 durch Einbringen eines stabförmigen Infrarotstrahlers 5, z.B. eines Nernst-Stiftes, oder dgl., ins Innere des Spritzenkörpers 1, wodurch die innere Oberfläche des gesamten Spritzenkörpers 1 auf Temperaturen oberhalb der maximalen Einsatztemperatur des Spritzenkörpers 1 aufgeheizt wird und somit der Gleitmittelfilm 4 thermisch fixiert wird.

Hierbei wird die Kopf-Geometrie des Infrarotstrahlers 5 so gewählt, daß nur eine minimale Abstrahlung in Richtung des Spritzenkopfes mit dem Luer-Konus 1b bzw. einer Kanüle und deren Verklebung im Spritzenkopf (nicht dargestellt) besteht.

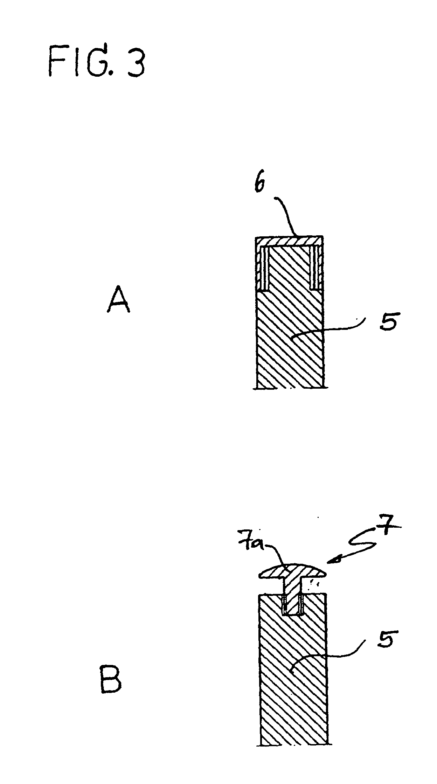

In Figur 2 ist diese Kopf-Geometrie durch die kopfseitige Abdeckscheibe 5a symbolisch angedeutet. Die Figur 3 zeigt dabei zwei typische Beispiele für die kopfseitige Ausbildung des stabförmigen Infrarotstrahlers 5. Im Figurenteil A ist auf dem Kopf des Infrarotstrahlers 5 eine zylindrische Abdeckkappe 6 aus entsprechend hitzefestem Material aufgeschraubt, während im Figurenteil B ein pilzförmiger Körper 7 in die Stirnseite des Kopfteiles 15 des Infrarotstahles 5 eingeschraubt ist, wobei der Schirm 7a des pilzförmigen Körpers 7 die Strahlung von dem Spritzenkopf fernhält.

Durch die Kopfgeometrie des Infrarotstrahlers gelingt es, den zylindrischen Teil des Spritzenkörpers 1 mit einer thermisch fixierten Gleitmittelschicht, z.B. Silikonöl, zu versehen, ohne daß es durch ausgehärtetes Silikonöl im Nadelkanal zu einer Verstopfung des Nadelkanals kommen kann.

Bei der Beschichtung von Glasspritzenkörpern mit eingeklebter Nadel können dabei Fixierungstemperaturen von ca. 300°C ohne Beeinträchtigung der Klebestelle erreicht werden, wobei sich zusätzlich mit Vorteil eine signifikante Reduzierung der Keimzahl und eine Reduktion von Endotoxinen ergibt.

Bei der Beschichtung von Kunststoff-Spritzenkörpern mit oder ohne integrierte Nadel können die Fixierungstemperaturen ca. 50°C oberhalb der maximalen Einsatztemperaturen des Kunststoff-Spritzenkörpers liegen. Bei der Fixierungserwärmung findet gleichzeitig (in situ) eine Oberflächenaktivierung statt, was sich vorteilhaft auf die Haftfestigkeit des Gleitmittelfilms auswirkt.

Das Gleitmittel besteht vorzugsweise aus Silikonölemulsion oder reaktiven Silikonölen, oder aus einer Mischung derselben entsprechend der eingangs zitierten DE 197 53 766 A1.

Reaktive Silikonöle enthalten im Gegensatz zu nicht-reaktiven Silikonölen reaktive funktionelle Gruppen, die eine Vernetzung in sich und ggf. auch mit anderen Oberflächen ermöglichen.

Der Aufbau des reaktiven Silikonöls ist gemäß seiner Funktion ein (Siloxan)-Molekül, welches je nach Aushärtungsmechanismus funktionelle Gruppen enthält. Ein reaktives Silikonöl enthält insbesondere Vinylgruppen, die unter dem Einfluß von Temperatur und UV-Bestrahlung vernetzen. Die Vernetzungsreaktion wird daher im speziellen von Platinverbindungen katalysiert.

Bei einem Gemisch aus reaktiven und nicht reaktiven Silikonöl bildet das ausgehärtete Silikonöl dann einen festen Film, der als Trennschicht zwischen Spritzenkörper und Kolbenstopfen fungiert. Ein Festbacken des insbesondere aus Brombutyl bestehenden Stopfens an der Oberfläche des Spritzenkörpers wird dadurch verhindert. Zur Herabsetzung der Gleittreibung wird dem reaktiven Silikonöl Dimethylpolysiloxane als nicht reaktives Silikonöl beigemischt, d.h., das anteilige reaktives Silikonöl bildet nach der Vernetzung praktisch die haftende Grundschicht, in die das anteilige nicht reaktive Silikonöl lokal eingebettet ist und die Gleitfähigkeit bewirkt.

Ein Silikonierungsmittel auf der Basis eines reaktiven Silikonöls setzt sich daher vorzugsweise zusammen aus; vinylgruppenhaltigem Silikonöl, Vernetzer, Katalysator, Lösungsmittel und nicht reaktivem Dimethylpolysiloxane.

Die Viskosität des reaktiven Silikonöles liegt bei Einkomponenten-Systemen im Bereich von 10 000 bis 100 000 mm²/s, bei Mehrkomponenten-Systemen im Bereich von 200 bis 5 000 mm²/s wogegen die Viskosität des nicht reaktiven Silikonöles im Bereich von 350 bis 20 000 mm²/s liegt.

Zum Auftragen der Silikonöle können diese noch verdünnt werden, insbesondere wenn das Auftragen durch Sprühen erfolgt.

Die Dicke der Silikon-Gleitschicht liegt vorzugsweise im Bereich von 10 nm bis 1 µm.

Die Silikonöle können aus den nachstehenden Silikonverbindungen bestehen:
Nicht vernetzte, polare Silikone oder Polysiloxane, insbesondere nicht reaktive Polydimethyldisiloxane (PDMS) oder reaktive Polymersiloxane, Copolymere von Alkylamin-modifizierten Methoxysiloxane, Polysiloxane mit einer Aminoalkyl-Gruppe.

Das Vernetzen erfolgt mit bekannten Methoden, insbesondere mit UV-Licht oder durch Plasmapolymerisation.

## Patentansprüche

1. Verfahren zum Aufbringen einer hitzefixierten Gleitmittelschicht auf die Innenwandung von zylindrischen medizinischen Behältern, die an einem Ende eine sich verjüngende Austrittsöffnung aufweisen, und die am anderen Ende mit einem im Behälter als Kolben verschiebbaren Elastomerstopfen verschließbar sind, mit den Schritten
- Auftragen eines hitzefixierbaren Gleitmittels auf die Innenwandung des Behälters mit bekannter Technologie,
- Vergleichmäßigen des aufgetragenen Gleitmittels zu einer Schicht, **dadurch gekennzeichnet, dass** des Hitzefixieren der Gleitmittelschicht durch eine Infrarotstrahlung selektiv im zylindrischen Mantelbereich des Behälters mit hohen Temperaturen oberhalb der maximalen betrieblichen Einsatztemperatur des Behälters, erfolgt.

2. Verfahren nach Anspruch 1, bei dem als Gleitmittel ein Silikonöl verwendet wird.

3. Verfahren nach Anspruch 2, bei dem als Silikonöl ein nicht reaktives Silikonöl oder ein reaktives Silikonöl oder eine Mischung aus beiden Silikonölen verwendet wird.

4. Verfahren nach einem der Ansprüche 1 bis 3, bei dem das Auftragen des hitzefixierbaren Gleitmittels durch Aufsprühen auf die Behälterinnenwand erfolgt.

5. Verfahren nach einem der Ansprüche 1 bis 4, bei dem das Hitzefixieren des Gleitmittels durch Infrarotstrahlung an der Innenwand eines Glasbehälters im Temperaturbereich von ca. 300°C erfolgt.

6. Verfahren nach einem der Ansprüche 1 bis 4, bei dem das Hitzefixieren des Gleitmittels durch Infrarotstrahlung an der Innenwand eines Kunststoffbehälters in einem Temperaturbereich erfolgt, der um ca. 50°C oberhalb der maximalen betrieblichen Einsatztemperatur des Kunststoffbehälters liegt.

7. Vorrichtung zum Durchführen des Verfahrens nach einem der Ansprüche 1 bis 6, mit
- einer Einrichtung zum Auftragen des hitzefixierbaren Gleitmittels (3) aus einem Vorratsbehälter auf die Innenwandung des Behälters (1),
- einer Einrichtung zum Vergleichmäßigen der aufgetragenen Gleitmittelschicht (4), und **gekennzeichnet durch**
- eine in das Innere des Behälters einbringbare stabförmige Infrarot-Strahlungsquelle (5), die kopfseitig eine Strahlungsabschirmung (5a, 6, 7) zum Fernhalten der Strahlung von der Austrittsöffnung des Behälters besitzt.

8. Vorrichtung nach Anspruch 7, mit einer Sprüheinrichtung (2) zum Auftragen des hitzefixierbaren Gleitmittels.

9. Vorrichtung nach Anspruch 7 oder 8, mit einer Abschirmung durch eine kopfseitige Abdeckung in Form einer zylindrischen Kappe (6).

10. Vorrichtung nach Anspruch 7 oder 8, mit einer Abschirmung in Form eines pilzförmigen Körpers (7), der mit seinem Stiel in der Kopfstirnseite befestigt ist und mittels seines Schirmes (7 a) die Infrarotstrahlung von der Austrittsöffnung des Behälters fernhält.

## Claims

1. A process for applying a heat-immobilized lubricant layer to the inner surface of cylindrical medicinal containers which have a tapering outlet opening at one end and which are closable at the other end by an elastomeric stopper slideable as a piston in the container, said process comprising the steps of
- applying a heat-immobilizable lubricant to the inner surface of the container using known technology,
- uniformizing the applied lubricant to form a layer, **characterized in that** the heat-immobilizing of the lubricant layer is effected by means of infrared radiation selectively in the cylindrical shell region of the container using high temperatures above the maximum in-service use temperature of the container.

2. A process according to claim 1, wherein the lubricant used is a silicone oil.

3. A process according to claim 2, wherein the silicone oil used is a non-reactive silicone oil or a reactive silicone oil or a mixture thereof.

4. A process according to any one of claims 1 to 3, wherein the applying of the heat-immobilizable lubricant is effected by spraying onto the container's inner surface.

5. A process according to any one of claims 1 to 4, wherein the heat-immobilizing of the lubricant is effected by infrared radiation at the inner surface of a glass container in the temperature region of about 300°C.

6. A process according to any one of claims 1 to 4, wherein the heat-immobilizing of the lubricant is effected by infrared radiation at the inner surface of a plastics container in a temperature range which is about 50°C above the maximum in-service use temperature of the plastics container.

7. Apparatus for carrying out the process according to any one of claims 1 to 6, having
- means for applying the heat-immobilizable lubricant (3) from a stock reservoir container to the inner surface of container (1),
- means for uniformizing the applied lubricant layer (4), and **characterized by**
- a rod-shaped infrared radiation source (5) which is insertable into the interior of the container and which possesses at the head end a radiation screen (5a, 6, 7) for keeping the radiation away from the container's outlet opening.

8. Apparatus according to claim 7, having spray means (2) for applying the heat-immobilizable lubricant.

9. Apparatus according to claim 7 or 8, having a screen due to a head-end covering in the form of a cylindrical hood (6).

10. Apparatus according to claim 7 or 8, having a screen in the form of a mushroom-shaped body (7) which is secured by its stem to the head face surface and utilizes its cap (7a) to keep the infrared radiation away from the outlet opening of the container.

## Revendications

1. Procédé pour appliquer une couche de lubrifiant fixée par la chaleur sur la paroi interne de récipients médicaux cylindriques, qui présentent en une extrémité une ouverture de sortie qui se rétrécit et qui peuvent être fermés en l'autre extrémité par un bouchon en élastomère pouvant être déplacé dans le récipient en tant que piston, comprenant les étapes
- application d'un lubrifiant pouvant être fixé par la chaleur sur la paroi interne du récipient par une technologie connue,
- uniformisation du lubrifiant appliqué en une couche, **caractérisé en ce que** la fixation par la chaleur de la couche de lubrifiant est réalisée par un rayonnement infrarouge sélectivement dans la zone de l'enveloppe cylindrique du récipient à des températures élevées supérieures à la température d'utilisation opérationnelle maximale du récipient.

2. Procédé selon la revendication 1, dans lequel on utilise une huile de silicone comme lubrifiant.

3. Procédé selon la revendication 2, dans lequel on utilise comme huile de silicone une huile de silicone non réactive ou une huile de silicone réactive ou un mélange des deux huiles de silicone.

4. Procédé selon l'une quelconque des revendications 1 à 3, dans lequel l'application du lubrifiant pouvant être fixé par la chaleur est réalisée par pulvérisation sur la paroi interne du récipient.

5. Procédé selon l'une quelconque des revendications 1 à 4, dans lequel la fixation par la chaleur du lubrifiant par rayonnement infrarouge sur la paroi interne d'un récipient en verre est réalisée dans la plage de température allant jusqu'environ 300°C.

6. Procédé selon l'une quelconque des revendications 1 à 4, dans lequel la fixation par la chaleur du lubrifiant par rayonnement infrarouge sur la paroi interne d'un récipient en matériau synthétique est réalisée dans une plage de température qui est supérieure d'environ 50°C à la température d'utilisation opérationnelle maximale du récipient en matériau synthétique.

7. Dispositif pour réaliser le procédé selon l'une quelconque des revendications 1 à 6, comprenant
- un dispositif pour appliquer le lubrifiant (3) pouvant être fixé par la chaleur à partir d'un réservoir sur la paroi interne du récipient (1),
- un dispositif pour uniformiser la couche (4) de lubrifiant appliquée et **caractérisé par** une source de rayonnement infrarouge (5) en forme de tige, pouvant être introduite à l'intérieur du récipient, présentant sur sa tête (5a, 6, 7) une protection contre le rayonnement pour tenir le rayonnement à l'écart de l'ouverture de sortie du récipient.

8. Dispositif selon la revendication 7, présentant un dispositif de pulvérisation (2) pour appliquer le lubrifiant pouvant être fixé par la chaleur.

9. Dispositif selon la revendication 7 ou 8, avec une protection par un recouvrement de la tête en forme d'un capuchon cylindrique (6).

10. Dispositif selon la revendication 7 ou 8, avec une protection sous forme d'un corps (7) en forme de champignon qui est fixé avec son pied sur la face frontale de la tête et qui tient le rayonnement infrarouge à l'écart de l'ouverture de sortie du récipient au moyen d'un écran (7a).
